# EUROPEAN PATENT APPLICATION

(11) **EP 4 696 366 A1**
(43) Date of publication of application: **18.02.2026**
(21) Application number: 24814471.9
(22) Date of filing: 29.05.2024
(51) Int. Cl.: A61N 1/36, A61N 1/372

(54) **APPARATUS FOR STIMULATING PERIPHERAL NERVE**

(30) Priority: 31.05.2023 CN 202310640781
(71) Applicant: SL Medtech Lab, Shanghai 201203 (CN)
(72) Inventor: CHEN, Yi, Shanghai 201203 (CN); XIA, Xiang, Shanghai 201203 (CN)
(74) Representative: Zacco Sweden AB
(86) International application number: PCT/CN2024/095981
(87) International publication number: WO 2024/245274

(57) **Abstract**

The present application relates to an apparatus (100) for stimulating a peripheral nerve. The apparatus comprises: an implantable electrode assembly (102), which can be implanted in the body of a patient, and is configured to apply a stimulation signal to a nerve. The implantable electrode assembly (102) comprises: a stimulation electrode (102a), which is fixed to a predetermined position of the body of the patient and is adjacent to a nerve therein, and is configured to operably apply a stimulation signal to the nerve; and an electrode circuit (102b), which is coupled to the stimulation electrode (102a), and is configured to receive stimulation energy in the form of an electromagnetic field, convert the stimulation energy into the stimulation signal and provide the stimulation signal to the stimulation electrode (102a), wherein the stimulation energy is from the outside of the implantable electrode assembly (102). The apparatus further comprises: a stimulation energy generator (104), which can be worn outside the body of the patient, so as to provide stimulation energy to the implantable electrode assembly (102) by means of electromagnetic field coupling. The stimulation energy generator (104) comprises: a generator circuit (658), which is used for generating stimulation energy in the form of an electromagnetic field; and a power source (660), which is coupled to the generator circuit (658), and is configured to supply power to the generator circuit (658).

## Description

### RELATED APPLICATIONS

This application claims priority to Chinese patent application No. 202310640781.X, filed on May 31, 2023, entitled "DEVICE FOR STIMULATING PERIPHERAL NERVE", the disclosure of which is incorporated herein by reference in its entirety.

### TECHNICAL FIELD

The present application relates to the technical field of medical devices, and in particular to a device for stimulating a peripheral nerve.

### BACKGROUND

Implantable neurostimulation devices are widely used in the diagnosis, monitoring, and treatment of diseases, such as cardiac pacemakers, brain pacemakers, defibrillators, neurostimulators, or the like. Taking a neurostimulator as an example, a pulse generator disposed in a closed housing is connected to electrodes by extension wires, thereby transmitting electrical pulses generated by the pulse generator to electrodes arranged at specific positions to perform electrical stimulation at such positions. A typical application scenario of a neurostimulator is deep brain neurostimulation, which treats diseases such as Parkinson's syndrome, drug addiction, etc., by electrically stimulating the patient's brain.

In addition to the brain, some neurostimulation devices for stimulating peripheral nerves have also emerged, for example, for the treatment of neuropathic pain, overactive bladder, or some other diseases. Peripheral nerve stimulation, commonly known as PNS, requires the surgical placement of a small electrode next to the peripheral nerves to transmit electrical pulses to the peripheral nerves to achieve the neuromodulation effect. Conventional peripheral nerve stimulation devices generally require the implantation of many components into the human body, including electrodes, pulse generators, extension wires, etc. These components necessitate separate incisions for implantation, with the entire surgery typically requiring three or more incisions. In addition, these implants are usually large in size, especially, the implantation of the pulse generator requires a 4- to 5-centimeter incision in the upper chest. Larger wounds may also cause various adverse reactions in patients, such as infection and pain.

Therefore, there is a need for an improved device for stimulating peripheral nerves.

### SUMMARY

According to various embodiments of the present application, a device for stimulating a peripheral nerve is provided.

A device for stimulating a peripheral nerve includes:
an implantable electrode assembly capable of being implanted in a patient's body and configured to apply a stimulation signal to a nerve; wherein the implantable electrode assembly includes:
   a stimulation electrode secured to a predetermined location of the patient's body and adjacent to the nerve in the patient's body, the stimulation electrode being configured to operably apply the stimulation signal to the nerve; and
   an electrode circuit coupled to the stimulation electrode and configured to receive stimulation energy in a form of an electromagnetic field, convert the stimulation energy into the stimulation signal, and provide the stimulation signal to the stimulation electrode, wherein the stimulation energy comes from outside the implantable electrode assembly; and
a stimulation energy generator capable of being worn externally on the patient's body to provide the stimulation energy to the implantable electrode assembly via electromagnetic field coupling; wherein the stimulation energy generator includes:
   a generator circuit configured for generating the stimulation energy in the form of the electromagnetic field; and
   a power supply coupled to the generator circuit and configured to supply power to the generator circuit.

In an embodiment, the stimulation electrode includes:
an electrode sleeve configured as a sheath structure having a hollow channel, wherein the electrode sleeve is provided with an opening along a circumferential direction thereof to allow the nerve to enter the electrode sleeve via the opening and be accommodated in the hollow channel of the electrode sleeve; and
an electrode contact protruded from an inner surface of the electrode sleeve to make contact with the nerve.

In an embodiment, the electrode sleeve has overlapping portions in the circumferential direction thereof to allow for variations in a sectional diameter of the electrode sleeve.

In an embodiment, the stimulation electrode includes a plurality of electrode contacts. The plurality of electrode contacts are arranged along a length direction of the electrode sleeve.

In an embodiment, each of the electrode contacts in the stimulation electrode extends along the circumferential direction of the electrode sleeve.

In an embodiment, the implantable electrode assembly further includes:
a lead configured to connect the electrode sleeve and the electrode circuit to achieve coupling between the stimulation electrode and the electrode circuit.

In an embodiment, the implantable electrode assembly further includes:
an electrode circuit housing defining a cavity configured for accommodating the electrode circuit.

In an embodiment, the electrode circuit housing further includes:
a housing body having an opening; and
a feedthrough cover connected to the housing body at the opening, wherein the feedthrough cover is provided with a through hole to allow the electrode circuit to be led out from the electrode circuit housing.

In an embodiment, the housing body has an elongated shape. The housing body has a metal ring on a side where the opening is located.

In an embodiment, the implantable electrode assembly further includes:
an anchoring member configured to secure the electrode circuit housing inside the patient's body, wherein the anchoring member includes a butterfly clasp, which is configured to secure the electrode circuit housing to bones or muscles around a predetermined location of the patient's body.

In an embodiment, the implantable electrode assembly further includes:
an anchoring member configured to secure the electrode circuit housing inside the patient's body, wherein the anchoring member includes a binding wire configured to secure the electrode circuit housing to muscles around a predetermined location of the patient's body.

In an embodiment, the stimulation electrode includes a plurality of electrode contacts. The stimulation energy generator is further configured to provide the stimulation signal in a form of pulse to the plurality of electrode contacts sequentially or simultaneously via the electrode circuit.

In an embodiment, a maximum voltage amplitude of the stimulation signal in the form of pulse is in a range of 3 V to 12 V, a pulse width is in a range of 30 us to 240 us, and a pulse frequency is in a range of 0.1 Hz to 50 Hz.

In an embodiment, the stimulation energy generator further includes:
an indicator member, the stimulation energy generator being further configured to indicate alignment between the stimulation energy generator and the implantable electrode assembly when the stimulation energy generator is aligned with the implantable electrode assembly.

In an embodiment, the device further includes:
a sensing element configured to sense an abnormal state of the patient's body and generate a corresponding indication signal.

In an embodiment, the electrode circuit includes an electrode antenna.

In an embodiment, the device further includes:
an extracorporal controller configured to adjust the stimulation energy and provide the adjusted stimulation energy to the implantable electrode assembly via the electromagnetic field coupling.

In an embodiment, the stimulation energy generator further includes:
a generator housing configured to accommodate at least a portion of the generator circuit and the power supply; and
a patch removably coupled to the generator housing.

In an embodiment, the generator circuit includes a generator antenna disposed in the patch.

In an embodiment, the patch includes:
a connecting member configured to removably couple to the generator housing.

In an embodiment, the generator housing includes:
a generator housing upper portion and a generator housing lower portion that collectively define a cavity configured for accommodating at least a portion of the generator circuit and the power supply.

In an embodiment, the generator circuit includes:
a generator antenna disposed in the generator housing.

In an embodiment, the generator circuit includes:
a plurality of generator antennas, wherein at least one of the plurality of generator antennas is disposed in the generator housing, and another of the plurality of generator antennas is disposed in the patch; and the generator antenna in the patch is configured to relay an electromagnetic field coupling between the at least one of the generator antennas in the generator housing and an electrode antenna in the implantable electrode assembly.

In an embodiment, the implantable electrode assembly further includes:
a housing body defining a cavity configured for accommodating the electrode circuit; and
wherein the stimulation electrode includes:
   an electrode sleeve attached to the housing body and configured as a sheath structure having a hollow channel, wherein the electrode sleeve has an opening along a circumferential direction thereof to allow the nerve to enter the electrode sleeve via the opening and be accommodated in the hollow channel of the electrode sleeve, and
   an electrode contact protruded from an inner surface of the electrode sleeve to make contact with the peripheral nerve.

### BRIEF DESCRIPTION OF THE DRAWINGS

In order to illustrate the technical solutions in the embodiments of the present application or prior art more clearly, the drawings used for describing the embodiments or prior art will be described briefly. Apparently, the following described drawings are merely for the embodiments of the present application, and other drawings can be derived by those of ordinary skill in the art without any creative effort.
FIG. 1a and FIG. 1b are schematic views showing an application of a device 100 for stimulating a peripheral nerve according to an embodiment of the present application.
FIG. 2a shows a perspective view of an implantable electrode assembly according to an embodiment of the present application.
FIG. 2b shows a sectional view of a stimulation electrode of an implantable electrode assembly according to an embodiment of the present application.
FIG. 2c shows an exploded view of an electrode circuit and a housing thereof according to an embodiment of the present application.
FIG. 3a shows a perspective view of an implantable electrode assembly 310 according to an embodiment of the present application.
FIG. 3b shows a sectional view of the implantable electrode assembly 310 according to an embodiment of the present application.
FIG. 4 shows a schematic structural view of an implantable electrode assembly according to another embodiment of the present application.
FIG. 5a and FIG. 5b are schematic structural views showing an implantable electrode assembly according to another embodiment of the present application.
FIG. 6a and FIG. 6b show schematic structural views of a stimulation energy generator according to an embodiment of the present application.
FIG. 7a to FIG. 7g show various exemplary configurations of stimulation energy generators according to some embodiments of the present application.

### DETAILED DESCRIPTION

The technical solutions in the embodiments of the present application will be clearly and completely described below with reference to the accompanying drawings in the embodiments of the present application. Apparently, the embodiments described are only part of the embodiments of the present application, not all of the embodiments. Based on the embodiments in the present application, all other embodiments obtained by ordinary skills in the art without making creative efforts are within the scope of protection of the present application.

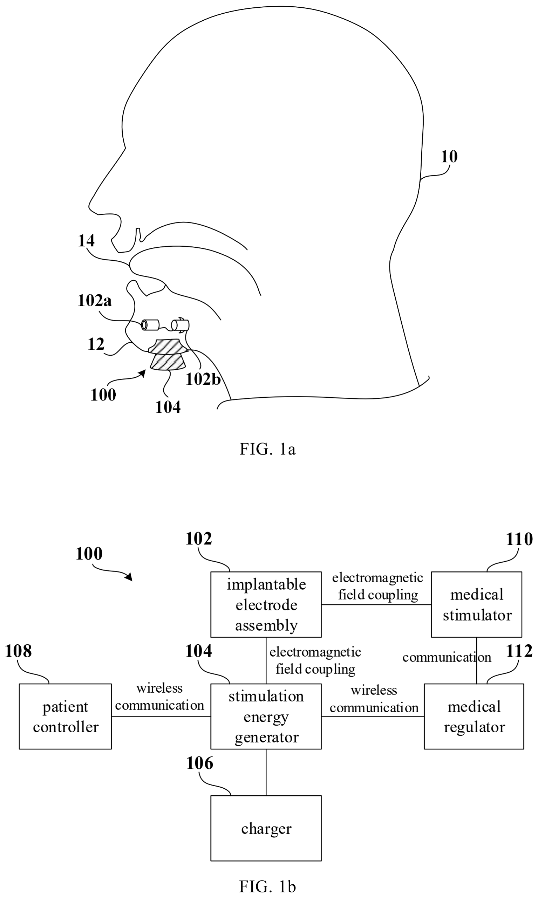

FIG. 1a and FIG. 1b show a device 100 for stimulating a peripheral nerve according to an embodiment of the present application. In an embodiment shown in FIG. 1a, a portion of the device 100 is positioned on a patient's head 10, for example, on the patient's mandible 12, and can be used to stimulate the hypoglossal nerve to treat Obstructive Sleep Apnea (OSA). During use, the device 100 can stimulate the patient's tongue 14 by stimulating one or more nerves in the hypoglossal nerve to cause a unidimensional forward extension or lifting motion of the patient's tongue 14, which increases the patient's respiratory ventilation, thereby avoiding or reducing apnea (temporary cessation of breathing) and/or hypopnea (slow or shallow breathing) during the patient's sleep.

It can be appreciated by those skilled in the art that the device for stimulating the peripheral nerve of the present application is not limited to the aforementioned application of hypoglossal nerve stimulation, but can also be used for various suitable diseases or indications. For example, the device can be used to stimulate the occipital nerve to treat neuropathic pain or migraine; can be used to stimulate the tibial nerve to treat overactive bladder; can be used to stimulate the vagus nerve to treat or improve epilepsy or depression; can be used to stimulate the phrenic nerve to treat respiratory insufficiency caused by phrenic nerve control disorders; and can also be used to stimulate the sacral nerve to treat urinary incontinence and various other neurogenic bladder diseases.

Taking sacral nerve stimulation as an example, sacral nerve stimulation can affect the actions of the bladder detrusor muscle and the external urethral sphincter, so that a suitable stimulation signal can be applied by a neurostimulation device to inhibit the hyperreflexia of the bladder detrusor muscle and achieve the purpose of alleviating urinary incontinence; optionally, the stimulation signal can adopt a specific stimulation pulse sequence, which can activate the coordinated actions of the bladder detrusor muscle and the external urethral sphincter to achieve controlled urination. When in use, an implantable electrode assembly of the sacral nerve stimulation device can be implanted in the patient's calf and adjacent to the sacral nerve, while the extracorporeal stimulation energy generator can be worn on the outer side of the calf, which can send and apply the pulse stimulation signal to the sacral nerve through the implantable electrode assembly, regulate the bladder and intestinal nerve reflexes related to urination and defecation, and restore the abnormal nerve reflexes to balance. Optionally, the patient can control the on/off switch and programmable settings of the stimulation energy generator through an external controller like a remote control, thereby starting or adjusting the nerve stimulation function as needed.

Unlike the central nervous system, the aforementioned various peripheral nerves are generally located outside the brain or spinal cord, with a thinner diameter and a wider and denser distribution of their terminal ends. To adapt to these peripheral nerves, the neurostimulation device according to the embodiments of the present application can adopt a smaller-sized and specially structured in-vivo stimulation electrode assembly, so that the stimulation electrode has a better fit with the peripheral nerve; in addition, the stimulation electrode generally adopts a sleeve structure, which allows the contacts of the stimulation electrode to be distributed circumferentially around the peripheral nerve, thereby more accurately transmitting the electrical stimulation signal to the designated nerve bundle and achieving refined regulation; the small-sized electrode structure allows it to be implanted and anchored near the peripheral nerve, so that a streamlined lead design can be adopted; in addition, the implantation near the peripheral nerve is usually close to the body surface (for example, less than 10 cm), so an extracorporeal wireless power supply method can be used to apply stimulation energy to the implanted electrode. Hereinafter, the neurostimulation device of the present application will be further illustrated by taking hypoglossal nerve stimulation as an example. However, it can be appreciated by those skilled in the art that that the neurostimulation device of the present application can be positioned in other parts of the patient's body in the same or similar manner, and stimulate the peripheral nerve(s) in these parts to treat corresponding diseases.

Still referring to FIG. 1a, the neurostimulation device 100 includes an implantable electrode assembly to be implanted in a patient' body. The implantable electrode assembly is generally positioned at a predetermined location within the patient' body (e.g., under the tongue), and configured to apply a stimulation signal to a nerve at the predetermined location. In an embodiment shown in FIG. 1a, the implantable electrode assembly has a split structure and includes a stimulation electrode 102a and an electrode circuit 102b. The stimulation electrode 102a and the electrode circuit 102b can be connected to each other via a lead. In some embodiments, the stimulation electrode 102a is configured as a cuff electrode that can enclose the outer side of a section of the peripheral nerve in a circumferential manner, thereby allowing one or more stimulation contacts on the outer side of the section of the peripheral nerve to operably apply a stimulation signal (e.g., a stimulating electrical pulse) to the peripheral nerve. The electrode circuit 102b is configured to receive stimulation energy in the form of an electromagnetic field from outside the patient's body, convert this stimulation energy into a stimulation signal, and provide the stimulation signal to the stimulation electrode 102a. Accordingly, the neurostimulation device 100 further includes a stimulation energy generator 104, which can be worn externally on the patient' body (e.g., on the skin surface on the outer side of the mandible 12) to provide stimulation energy to the implantable electrode assembly via electromagnetic field coupling. It should be noted that although FIG. 1a only shows that the implantable electrode assembly includes one stimulation electrode 102a, in some other embodiments, the implantable electrode assembly may also include another number of stimulation electrodes, such as two, three, or more, which are respectively disposed at different sections of the peripheral nerve that are substantially adjacent to each other and are coupled to each other via separate leads, or coupled to the electrode circuit 102b via separate leads. In this way, the electrode circuit 102b can provide corresponding stimulation signals to these stimulation electrodes 102a, such as applying stimulation signals simultaneously or applying stimulation signals in a time-division manner, and the applied stimulation signals can be the same or different.

FIG. 1b is a schematic block diagram of the neurostimulation device 100 according to an embodiment of the present application. As shown in FIG. 1b, in addition to the implantable electrode assembly 102 and the stimulation energy generator 104, the neurostimulation device 100 may further include some other components to enable configuration and in vitro control of the neurostimulation device 100. Optionally, the neurostimulation device 100 may include a charger 106 configured for charging the stimulation energy generator 104, especially when a rechargeable power supply is employed in the stimulation energy generator 104. For example, when the patient does not need neurostimulation treatment (for example, when the patient is not in a sleeping state), the stimulation energy generator 104 can be removed from the patient's body and connected to the charger 106 for charging.

In some embodiments, the neurostimulation device 100 may further include a patient controller 108. The patient controller 108 can wirelessly communicate with the stimulation energy generator 104 to monitor the operating status of the stimulation energy generator 104, for example, to monitor the power level of the power supply, or to allow the patient to configure some parameters of the operation of the stimulation energy generator 104, for example, to configure the maximum voltage amplitude, pulse width, pulse frequency, device operating time, etc. of the stimulation signal. It can be understood that in some cases, particularly when the stimulation energy generator 104 fails or is in an abnormal state, the patient controller 108 may also receive a corresponding alarm signal from the stimulation energy generator 104 and notify the patient of the failure or abnormal state. In some embodiments, the patient controller 108 may be a dedicated hardware device. In other embodiments, the patient controller 108 may also be implemented as software or an application that can run on a smartphone, tablet computer, or other similar general-purpose hardware device, and utilize the software and hardware processing capabilities of these general-purpose hardware devices to implement the functions of the patient controller. For example, the stimulation energy generator 104 can be integrated with a Bluetooth module or a communication module that complies with a similar wireless communication protocol, or integrated with a communication module that complies with a USB, Type-C protocol or a similar wired communication protocol, so that it can communicate with the patient controller 108 or similar devices through these communication modules.

In some embodiments, the neurostimulation device 100 may further include a medical stimulator 110 and a medical regulator 112, which, for example, can be operated by a doctor to configure or control the operation of the implantable electrode assembly 102 and the stimulation energy generator 104. In particular, during the surgery performed by the doctor to place the implantable electrode assembly 102 into the patient's body, the medical stimulator 110 can temporarily replace the stimulation energy generator 104 to electromagnetically couple with the implantable electrode assembly 102 and provide stimulation energy to it. In this way, during the surgery, the doctor can assess the placement position of the implantable electrode assembly 102 and whether the contact between the electrode and the peripheral nerve(s) is effective, and can adjust the position of the implantable electrode assembly 102 in the body in a timely manner to achieve the optimal implantation effect. The medical regulator 112 can communicate with the medical stimulator 110 in a wireless or wired manner to control the medical stimulator 110, for example, to configure the maximum voltage amplitude, pulse width, pulse frequency, etc. of the stimulation signal to be applied during surgery or during patient's routine follow-up. In some embodiments, the medical regulator 112 and the medical stimulator 110 can be used for routine follow-ups of the patient, usually 1 week, 2 weeks, 4 weeks, etc. after the implantation surgery. This process is also called "titration". During the follow-up process, the doctor can perform titration again one or more times. During each titration process, the medical regulator 112 can automatically analyze the patient's historical data and establish a lookup table of the linear relationship between the neural interface impedance, the stimulation threshold voltage, and the stimulation parameters. After calculating the new neural interface impedance, the parameters of the stimulation signal to be adjusted can be obtained by querying the lookup table. The medical regulator 112 can be configured in the stimulation energy generator 104 for daily treatment of the patient. It can be understood that the medical regulator 112 can be used in conjunction with the medical stimulator 110 (which is usually a desktop device rather than a wearable device), but it can also be used in conjunction with the stimulation energy generator 104, for example, in a manner similar to that of the patient controller 108.

**In** some embodiments, an extracorporeal controller, such as patient controller 108 or medical regulator 112, used outside the patient's body, not only can adjust stimulation energy, but also can receive a physiological signal from the patient and adjust the stimulation energy to be applied to the patient based on changes in the patient's physiological signal. For example, the patient's physiological signal can be a change in the impedance of a portion of the patient's body tissue (e.g., tissue adjacent to the peripheral nerve(s) to be stimulated) sensed and collected by the implantable electrode assembly. To adapt to certain impedance changes, for example, when impedance decreases, the voltage amplitude of the stimulation signal applied to the peripheral nerve(s) can be reduced to avoid excessive current damage to the patient's body tissue. For another example, when impedance increases, the voltage amplitude of the stimulation signal applied to the peripheral nerve(s) can be increased to meet the minimum threshold requirement for nerve stimulation, preventing the therapeutic effect from being unable to achieve due to ineffective nerve stimulation. In some other examples, the patient's physiological signals can also be detected by additional sensing elements disposed inside or outside the patient's body. These additional sensing elements can be communicatively coupled to the stimulation energy generator 104 to provide it with the detected patient physiological signals. For example, the sensing element can be used to detect the patient's breathing rhythm, or the movement of the patient's external urinary sphincter, etc. These physiological signals can be used to adjust the stimulation duration. Generally speaking, when muscle control is required, the stimulation duration can be minimized to better protect the patient's tissue from damage caused by prolonged stimulation.

As mentioned above, in some embodiments, the neurostimulation device 100 may further include the sensing element, which may be integrated into the implantable electrode assembly or the stimulation energy generator, or may be constructed as a separate component (which may be placed on the patient's body). For hypoglossal nerve stimulation applications, the sensing element may sense an abnormal state of the patient's tongue (e.g., abnormal conditions of the tongue muscles or nerve tissue) and generate a corresponding indication signal. In some embodiments, the sensing element may be an accelerometer that detects abnormal conditions such as apnea or hypopnea that occur during the patient's sleep by detecting the movement of the patient's head. Accordingly, based on the physiological signal reflecting the abnormal condition generated by the sensing element (e.g., when the patient is breathing abnormally), the stimulation energy generator may be allowed to apply stimulation energy to the patient; whereas in other cases (e.g., when the patient is breathing normally), the stimulation signal may not be applied.

It will be appreciated that FIG. 1b merely exemplarily illustrates some components of the neurostimulation device 100 according to some embodiments of the present application. Aside from the implantable electrode assembly 102 and the stimulation energy generator 104, the other components are not essential. A doctor, patient, or other personnel may select one or more of these components based on practical circumstances.

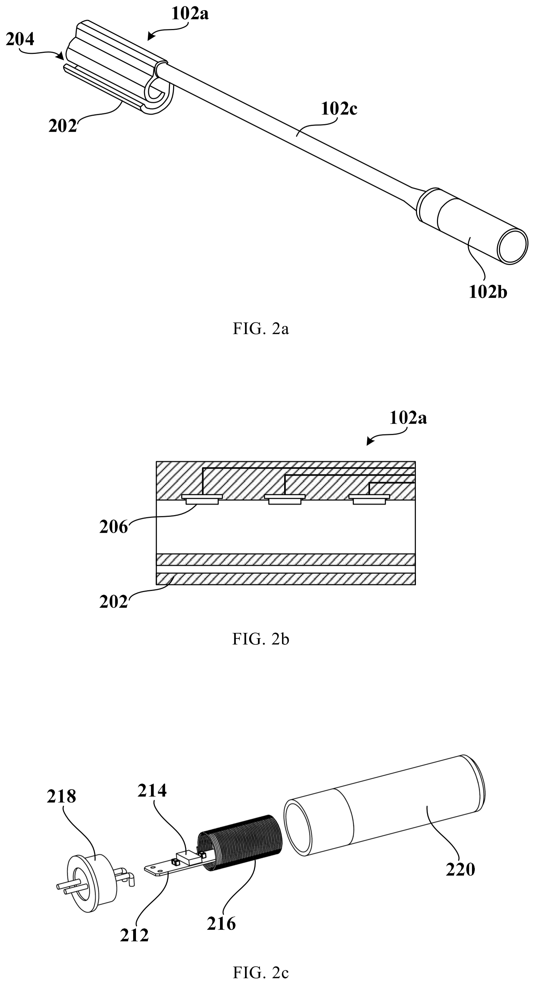

FIGS. 2a to 2c illustrate an example of the implantable electrode assembly shown in FIG. 1a, where FIG. 2a is a perspective view of the implantable electrode assembly, FIG. 2b is a sectional view of the stimulation electrode of the implantable electrode assembly, and FIG. 2c is an exploded view of an electrode circuit and a housing thereof.

As shown in FIG. 2a, the implantable electrode assembly includes a stimulation electrode 102a and an electrode circuit 102b, which are coupled to each other via a lead 102c. In some embodiments, the lead 102c can be made of a flexible material (e.g., silicone), with a conductive wire encapsulated therein. This allows the spacing between the stimulation electrode 102a and the electrode circuit 102b to be adjusted to some extent to accommodate securing requirements at different positions.

Referring to FIGS. 2a and 2b, the stimulation electrode 102a includes an electrode sleeve 202. The electrode sleeve 202 is configured as a sheath structure having a hollow channel. The electrode sleeve 202 is provided with an opening 204 along its circumferential direction to allow a nerve to enter the electrode sleeve 202 via the opening 204 and be contained within the hollow channel of the electrode sleeve 202. In some embodiments, the electrode sleeve 202 can be made of a flexible material, or optionally a biocompatible flexible material, such as silicone or polyurethane, which can provide electrode securing function while avoiding damage to the patient's nerves or other adjacent tissues. In some embodiments, the electrode sleeve 202 has a size comparable to that of the peripheral nerve to be stimulated. For example, in a relaxed state, the diameter of the electrode sleeve 202 is approximately 50% to 98%, and optionally 80% to 95%, of the diameter of the peripheral nerve. Thus, when implanted in a patient and enclosed around the peripheral nerve, the electrode sleeve 202 can slightly expand outwardly and generate pressure towards the peripheral nerve, which allows the electrode sleeve 202 to be more securely secured to the peripheral nerve and prevent it from sliding on the peripheral nerve due to the patient's body movements. In some embodiments, the electrode sleeve 202 has overlapping portions in its circumferential direction to allow for variations in the sectional diameter of the electrode sleeve. As such, even if the electrode sleeve 202 slightly expands outwardly due to the accommodation of the peripheral nerve therein, the electrode sleeve 202 can still substantially completely enclose the nerve without forming a significant circumferential gap, thereby ensuring sufficient contact between the electrode sleeve 202 and the peripheral nerve.

The stimulation electrode 102a further includes an electrode contact 206 that is protruded from the inner surface of the electrode sleeve 202 to contact the nerve. In some embodiments, the electrode sleeve 202 can have a certain length so that when it is attached to the peripheral nerve, the electrode contact 206 can extend a certain length along the length direction of the peripheral nerve. The stimulation electrode 102a can also include a plurality of electrode contacts 206, for example three electrode contacts 206 as shown in FIG. 2b, which are arranged along the length direction of the electrode sleeve 202. The plurality of electrode contacts 206 not only can apply stimulation signals alternately to provide more possible treatment options, but also can increase the contact between the stimulation electrode 102a and the peripheral nerve, further improving the stability and reliability of the use of the neurostimulation device. Alternatively, each electrode contact in the stimulation electrode can also extend along the circumferential direction of the electrode sleeve 202, or extend in a spiral manner in the electrode sleeve 202. It can be understood that the distribution and configuration of the electrode contacts 206 can be configured according to the sensitive section of the peripheral nerve to be stimulated.

In some embodiments, the stimulation electrode includes a plurality of electrode contacts, and the stimulation energy generator can sequentially provide stimulation energy in the form of pulses to the plurality of electrode contacts through the electrode circuit, where the electrode circuit can convert the stimulation energy into stimulation signals in the form of pulses in sequence. In other embodiments, the stimulation energy generator can also simultaneously provide stimulation energy in the form of pulses to the plurality of electrode contacts, where the electrode circuit can convert the stimulation energy into stimulation signals in the form of pulses. Optionally, the maximum voltage amplitude of the stimulation signal in the form of pulses is in a range of 3V to 12V, the pulse width is in a range of 30us to 240us, and the frequency of the stimulation pulses is in a range of 0.1Hz to 50Hz.

Still referring to FIG. 2a, the electrode circuit 102b is housed in an electrode circuit housing. The electrode circuit housing can have a sealed design to encapsulate the electrode circuit 102b therein and prevent liquids from penetrating through the housing to the electrode circuit 102b and affecting or disrupting the normal operation of the electrode circuit 102b. The structure of the electrode circuit 102b and its electrode circuit housing will be further described below. With the aid of the electrode circuit housing, the electrode circuit 102b can be implanted in the patient's body and adjacent to the stimulation electrode 102a. In some embodiments, the electrode circuit housing can be secured into the patient's body using an anchoring member to enhance the stability and securement of the electrode circuit 102b within the body. In some embodiments, the anchoring member can include a butterfly clasp configured to secure the electrode circuit housing to bone or muscle around a predetermined location in the patient's body. The butterfly clasp has through-holes on both sides for a screw to pass through and drill into the bone, thereby securing the electrode circuit housing to the bone. Optionally, the through-holes in the butterfly clasp can also serve as securing holes for surgical sutures. After passing through the through-holes, the sutures are secured to the muscle tissue at both ends, thereby securing the implant to the muscle. In other embodiments, the anchoring member comprises a binding wire, which is configured to secure the electrode circuit housing to the muscle around a predetermined location on the patient's body. The outer surface of the electrode circuit housing may be provided with a groove. The binding wire can be secured into the groove by winding. Two ends of the binding wire can be sutured into the muscle tissue, thereby securing the electrode circuit housing to the muscle. It can be understood that various suitable anchoring members can be used to secure the electrode circuit and its housing. In some embodiments, the anchoring member can be made of a biodegradable material. The anchoring structure can be made of a metal material or a polymer material; the metal material can be a titanium alloy; the polymer material can be an absorbable material such as polycaprolactone (PCL), poly(lactic-co-glycolic acid) (PLGA), poly(DL-lactic acid) (PDLLA), poly(L-lactic acid) (PLLA), or polydioxanone (PDO). These materials can be absorbed by human tissue during the healing process, further minimizing tissue damage and enhancing patient recovery.

Referring to FIG. 2c, FIG. 2c shows a schematic view of the electrode circuit and its housing. In some embodiments, the electrode circuit may include a circuit substrate 212. Various electronic components 214 (e.g., passive components such as capacitors, resistors, and switches, or active components such as amplifiers and transistors) that constitute the electrode circuit may be mounted on the circuit substrate 212 and interconnected to one another. In addition, the electrode circuit further includes an electrode antenna 216. The electrode antenna 216 is electrically coupled to the electronic components 214 and optionally mounted on the circuit substrate 212, to receive stimulation energy in the form of an electromagnetic field from the outside and generate induced electric potential energy. This induced electric potential energy can be converted into a stimulation signal by the electrode circuit formed by the electronic components 214, and provide the stimulation signal to an output terminal of the electrode circuit. The output terminal can be coupled to the electrode contact via a conductive wire, so that the stimulation signal can be applied to the patient's peripheral nerve via the electrode contact. It can be appreciated by those skilled in the art that the electrode circuit is a circuit that configured to receive wireless electromagnetic field energy and converts it into a pulse signal in a predetermined form. Any suitable circuit design can be used to implement the electrode circuit, and detailed description thereof is not provided herein. In some embodiments, the electrode antenna 216 may employ a helical antenna design. Due to its compact structure, the helical antenna is suitable for reducing the volume of the electrode circuit and its housing.

The electrode circuit housing may include a housing body 220 having an opening at one end to allow components such as the circuit substrate 212 and the electrode antenna 216 to be placed within the housing body 220. The electrode circuit housing further includes a feedthrough cover 218. The feedthrough cover 218 is connected to the housing body 220 at the opening of the housing body 220. The feedthrough cover 218 has a through hole to allow the electrode circuit (e.g., an output terminal of the electrode circuit) to be led out of the electrode circuit housing. In some embodiments, the housing body 220 includes an electrode antenna, for example in a shape of a hollow cylinder as shown in FIG. 2c. The housing body 220 has a metal ring on the side where the opening is located, to serve as a buffer and connection area between the housing body 220 and the feedthrough cover 218. In some embodiments, in addition to the metal ring, the housing body 220 and the feedthrough cover 218 may be formed of a dielectric material such as ceramic or a polymer. Such material can provide sufficient electrical isolation while maintaining airtightness, without affecting the electrode antenna 216's ability to receive the energy of an external electromagnetic field.

As previously mentioned, for the implantable electrode assembly shown in FIG. 2a, the stimulation electrode 102a and the electrode circuit 102b can be coupled using the lead 102c. This allows the induction of the electromagnetic field and the application of the stimulation signal to be implemented at different locations, thereby increasing the flexibility of the configuration of the implantable electrode assembly. However, in some alternative embodiments, the implantable electrode assembly may also use an integrated stimulation electrode and electrode circuit, that is, the stimulation electrode and the electrode circuit are encapsulated in the same housing without being connected to each other via the lead. This helps to simplify the structure of the implantable electrode assembly and reduce the complexity of surgical placement of the implantable electrode assembly in the patient's body.

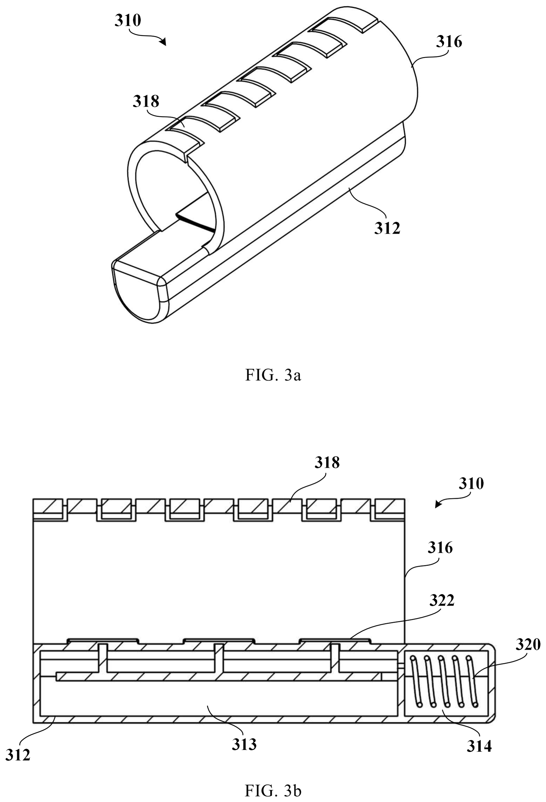

FIGS. 3a and 3b illustrate an implantable electrode assembly 310 according to an embodiment of the present application, which adopts an integrated structure. FIG. 3a is a perspective view of the implantable electrode assembly 310, while FIG. 3b is a sectional view of the implantable electrode assembly 310. In some examples, the implantable electrode assembly 310 can be placed near a patient's peripheral nerve and close to the body surface, enabling it to receive electromagnetic field energy from the outside and apply corresponding stimulation energy to the peripheral nerve.

As shown in FIGS. 3a and 3b, the implantable electrode assembly 310 includes a housing body 312. The housing body 312 is configured to house an electrode circuit (not shown) and an electrode antenna 320. The housing body 312 can have an elongated shape, such as a truncated cylinder or a prism. Optionally, the housing body 312 can include a plurality of cavities, for example, a circuit cavity 313 and an antenna cavity 314 shown in FIG. 3b. The electronic components and substrate of the electrode circuit can be placed in the circuit cavity 313, while the electrode antenna 320 can be placed in the antenna cavity 314. The electrode antenna 320 can be coupled to the electrode circuit via a conductive wire (not shown). Optionally, the circuit cavity 313 can be partially made of metal or a similar material, for example, its inner wall can be covered with a layer of metal material, which can improve the shielding performance of the circuit cavity 313 against external electromagnetic waves. The antenna cavity 314 does not have such electromagnetic shielding material, thereby allowing the electrode antenna 320 to receive electromagnetic field energy from the outside. In some alternative embodiments, the housing body 312 may define only one cavity for accommodating the electronic components, substrate, and electrode antenna 320. The housing body 312 may be made of various suitable materials, such as biocompatible metals (e.g., titanium alloys), polymers (e.g., polyimide, polyester resins), ceramic materials (e.g., zirconium oxide or aluminum oxide ceramic materials), or other suitable materials. It can be appreciated that in some embodiments, the plurality of cavities within the housing body 312 may be separately machined and subsequently connected together (e.g., by welding, bonding, riveting, or other connection methods), or may be integrally formed.

The implantable electrode assembly 310 further includes an electrode sleeve 316. The electrode sleeve 316 is configured in a generally hollow cylindrical shape and extends along the length direction of the housing body 312. The electrode sleeve 316 may have an opening that divides the electrode sleeve 316 into two sections. Each of the two sections is secured to the housing body 312 at one end and extends away from the housing body 312 to the opening, thereby allowing the peripheral nerve to pass through the opening and be received in the electrode sleeve 316. In an embodiment shown in FIGS. 3a and 3b, each of the two sections of the electrode sleeve 316 has a comb-like edge 318 adjacent to the opening, the two comb-like edges 318 can be engaged with each other to securely secure the electrode sleeve 316 to the peripheral nerve and allow the diameter of the electrode sleeve 316 to vary slightly to accommodate peripheral nerves of varying diameters. The number of engaging teeth on each comb-like edge 318 can be equal or unequal. In some embodiments, the length of the electrode sleeve 316 is shorter than the length of the housing body 312, thereby allowing the antenna cavity 314 of the housing body 312 to extend beyond the electrode sleeve 316. In this way, the electrode antenna 320 received in the antenna cavity 314 may not be blocked by the electrode sleeve 316, and can therefore better receive external electromagnetic field energy. In some embodiments, the electrode sleeve 316 can be a flexible material, or a flexible material with biocompatibility, such as silicone, polyurethane material, etc.; for example, the electrode sleeve 316 can be connected to the housing body 312 by injection molding, bonding, or melting. In some optional embodiments, the electrode sleeve 316 can extend beyond the housing body 312, for example, extend beyond the housing body 312 at its end away from the antenna cavity 314. In this way, the length of the electrode sleeve 316 is not limited by the length of the housing body 312, and the electrode sleeve 316 can extend to a sufficient length according to the length of a section of the peripheral nerve to be stimulated, to ensure sufficient and effective contact with the peripheral nerve.

On the inner surface of the electrode sleeve 316 adjacent to the housing body 312, a plurality of electrode contacts 322 are protruded such that they can make contact with a section of the peripheral nerve enclosed by the electrode sleeve 316. The electrode contacts 322 are generally distributed along the extending directions of the electrode sleeve 316 and the housing body 312 to increase the contact length between the peripheral nerve and the implantable electrode assembly 310. These electrode contacts 322 can be coupled to the electrode circuit in the circuit cavity 313 of the housing body 312 through conductive wires to receive stimulation signals therefrom. Although in FIG. 3b, the electrode contact 322 only occupies a small section of the circumferential length of the electrode sleeve 316, in some other embodiments, the electrode contact 322 can also extend a longer length along the circumferential direction of the electrode sleeve 316, for example, generally occupying 20%, 40%, 60%, 80%, 90% or more of the circumference of the electrode sleeve 316.

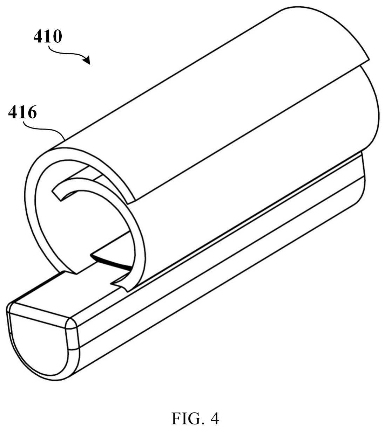

FIG. 4 shows an implantable electrode assembly 410 according to another embodiment of the present application. The implantable electrode assembly 410 adopts an integrated structure similar to the implantable electrode assembly 310 in FIGS. 3a and 3b, except that the opening of the electrode sleeve 416 does not adopt a comb-like edge, but instead adopts a structure with overlapping edges similar to the electrode sleeve 202 in FIG. 2a.

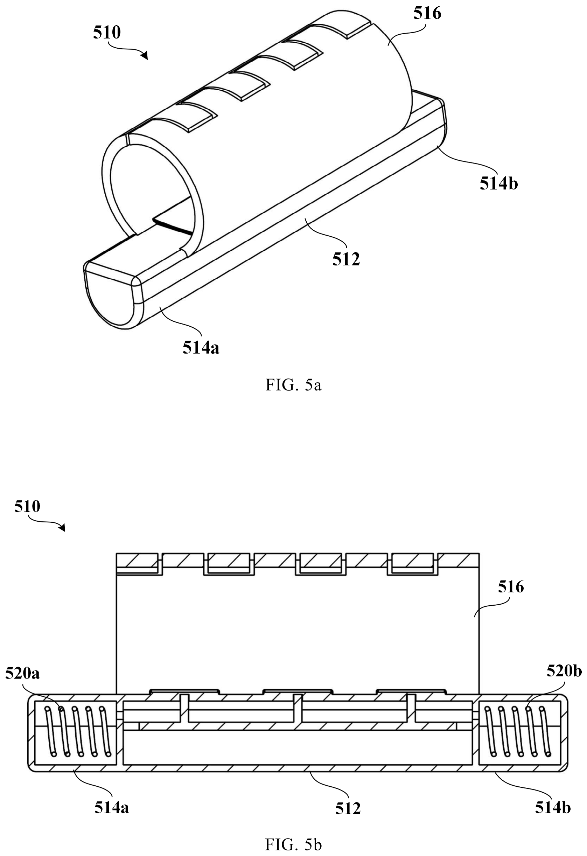

FIGS. 5a and 5b show an implantable electrode assembly 510 according to another embodiment of the present application, which also adopts an integrated structural configuration. FIG. 5a is a perspective view of the implantable electrode assembly 510, and FIG. 5b is a sectional view of the implantable electrode assembly 510. Different from the single-sided electrode antenna of the implantable electrode assembly 310 in FIGS. 3a and 3b, the implantable electrode assembly 510 shown in FIGS. 5a and 5b adopts a double-sided electrode antenna, that is, at both ends of an electrode shell 512 in an extending direction thereof, two electrode antennas 520a and 520b are respectively accommodated in corresponding antenna cavities 514a and 514b, and these two antennas 520a and 520b do not substantially overlap with the electrode sleeve 516. The use of two electrode antennas can improve the ability of the implantable electrode assembly 510 to receive external electromagnetic field energy. In an embodiment shown in FIG. 5b, the winding directions of the two electrode antennas 520a and 520b are substantially the same; in some alternative embodiments, the winding directions of the two electrode antennas 520a and 520b may also be different from each other, for example, perpendicular to each other or disposed at other angles to each other, which can reduce the placement requirements of the implantable electrode assembly 510 in the patient's body, especially the orientation requirements relative to the extracorporal energy generator.

Returning to FIG. 1a, in conjunction with the implantable electrode assembly, the stimulation energy generator 104 is worn externally on the patient's body to provide stimulation energy to the implantable electrode assembly in the patient's body through electromagnetic field coupling. Since it needs to be worn externally on the patient's body, for example, in the form of a patch, the stimulation energy generator 104 can adopt a split structure. That is, the stimulation energy generator 104 includes a patch for wearing and usually disposable, and a generator housing configured for accommodating the electronic components of the stimulation energy generator and usually reusable. In this way, at least a portion of the components of the stimulation energy generator 104 can be reused, thereby saving usage costs. In some embodiments, the stimulation energy generator 104 may include a generator circuit configured for generating stimulation energy in the form of an electromagnetic field, and a power supply coupled to the generator circuit and configured to supply power to the generator circuit. At least a portion of the generator circuit and the power supply can be accommodated in the generator housing.

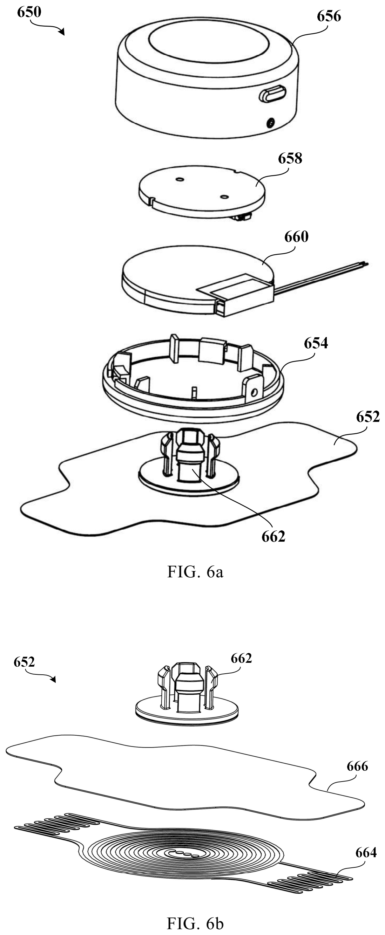

FIGS. 6a and 6b show an example of a stimulation energy generator 650 according to an embodiment of the present application. The stimulation energy generator 650 adopts a split structure.

Optionally, as shown in FIG. 6a, the stimulation energy generator 650 includes a patch 652 that can be affixed to the patient's body surface to secure the stimulation energy generator 650 externally on the patient's body, typically at a position near the implantable electrode assembly. The stimulation energy generator 650 further includes a generator circuit housing. In FIG. 6a, the generator circuit housing includes a generator circuit housing lower portion 654 and a generator circuit housing upper portion 656. The generator circuit housing lower portion 654 and the generator circuit housing upper portion 656 collectively define a cavity for accommodating at least a portion of a generator circuit 658 and a power supply 660. The generator circuit 658 is a primary controller of the stimulation energy generator 650 and is primarily configured for generating the stimulation energy required for peripheral nerve stimulation. Optionally, the generator circuit 658 may also have other functions, such as turning the stimulation energy generator 650 on and off and displaying its status. A display module (e.g., an LCD or LED display) may be mounted on the generator circuit 658. During use, the patient can observe the display module on the generator circuit 658 through a light-transmissive window on the generator circuit housing upper portion 656 to learn the operating status of the stimulation energy generator 650.

To transmit electromagnetic fields towards the implantable electrode assembly within the body, the stimulation energy generator further includes a generator antenna 664. In an example shown in FIGS. 6a and 6b, the generator antenna 664 is disposed on the patch 652, for example, attached to or sandwiched within a patch layer 666 (only one patch layer 666 is shown, but two or more patch layers may be used in practice). The patch layer 666 may be made of, for example, a hydrogel, a fabric material, or the like. Accordingly, the generator circuit 658, located within the generator circuit housing, does not include the generator antenna 664 but is instead coupled to the generator antenna 664 via a conductive wire. Optionally, the generator circuit 658 may be coupled to the generator antenna 664 via an electrical interface located at the generator circuit housing lower portion 654 and a connecting member 662 located on the patch 652. The connecting member 662 can be connected to the generator circuit housing lower portion 654, for example, in a snap-fit manner, thereby allowing the patch 652 to be removably coupled to the generator circuit housing and establishing an electrical connection between the generator circuit 658 and the generator antenna 664. In this way, stimulation energy from the generator circuit 658 can be radiated into the space surrounding the generator antenna 664 in the form of an electromagnetic field, so that it can be coupled and received by the implantable electrode assembly. In some embodiments, the power supply 660 can be a disposable battery. When its charge is depleted, this disposable battery can be replaced. In alternative embodiments, the power supply 660 can also be a rechargeable battery, and the generator circuit 658 can be integrated with a charging control module and functionality to control the charging of the power supply 660, or it can be charged by an external charger. Optionally, the generator antenna 664 can also be compatible with existing wireless charging technology, so that the power supply 660 can be charged by receiving external energy through the generator antenna 664.

In the patch 652 shown in FIG. 6b, the generator antenna 664 employs a planar antenna coil, and the generator antenna 664 is not disposed at other positions of the stimulation energy generator 650; however, it can be understood that in some other embodiments, the stimulation energy generator 650 may also adopt other antenna coil settings.

To ensure efficient coupling between the generator antenna 664 and the electrode antenna in the implantable electrode assembly, the distance between the two antennas can be in a range of 0.5 cm to 5 cm. In some embodiments, to measure and determine the coupling efficiency between the extracorporal and intracorporal antennas, the stimulation energy generator can further include an indicator member. The indicator member is capable of indicating alignment between the stimulation energy generator and the implantable electrode assembly when the stimulation energy generator is aligned with the implantable electrode assembly. For example, the stimulation energy generator can include a circuit module that detects electromagnetic coupling. When a change in the position between the two antennas causes a change in the degree of electromagnetic coupling, the circuit module can generate a corresponding coupling detection signal. As such, when the coupling detection signal indicates that the electromagnetic coupling between the antennas meets predetermined requirements, the indicator member can generate a corresponding alignment prompt signal, for example, in the form of vibration, sound, or light. This makes it more convenient for the patient to wear the extracorporal stimulation energy generator and avoids the impact of poor alignment between the antennas on the delivery of stimulation energy. It can be understood that in some cases, particularly when the electromagnetic coupling between the antennas does not meet predetermined requirements, the indicator member can continuously generate a corresponding alignment prompt signal, for example, by continuous vibration. This can alert the patient to poor alignment between the antennas and the need to adjust his posture or the position of the stimulation energy generator to meet the antenna alignment requirements. For example, when a patient is sleeping, if excessive rotation of the patient's head causes misalignment between the antennas of the stimulation energy generator and the implantable electrode assembly, the indicator member may vibrate continuously to alert the patient to adjust his sleeping posture.

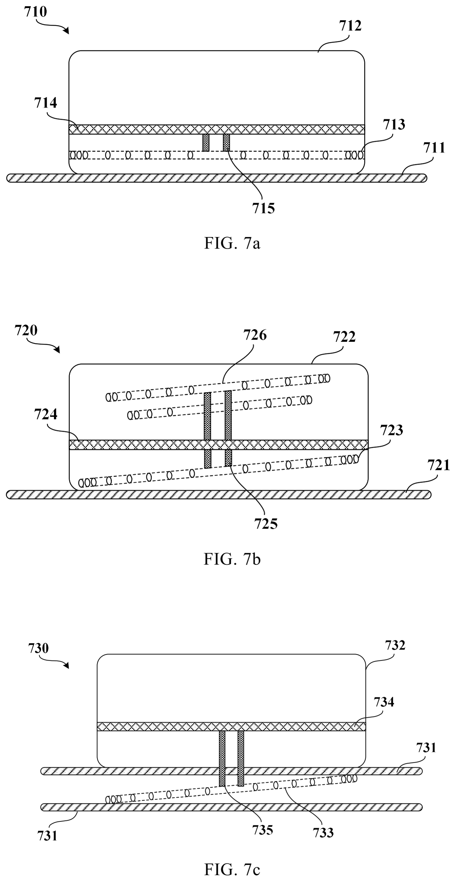

FIGS. 7a to 7g illustrate various exemplary configurations of stimulation energy generators according to some embodiments of the present application, which adopt configurations different from those shown in FIGS. 6a and 6b.

As shown in FIG. 7a, a stimulation energy generator 710 adopts a split configuration, and includes a patch 711 and a generator housing 712 coupled to the patch 711. A generator antenna 713 and a generator circuit 714 are both disposed inside the generator housing 712 and are coupled to each other by a conductive wire 715. In this way, the patch 711 mainly acts as a securing mechanism and may not include electronic components, thereby further increasing the number of reusable components and reducing the cost of use. Optionally, the generator antenna 713 can adopt the configuration of a planar antenna coil. In this case, the generator antenna 713 can be disposed as close as possible to the bottom of the generator housing 712 to reduce its distance from the electrode antenna in the body.

As shown in FIG. 7b, a stimulation energy generator 720 adopts a split configuration, and includes a patch 721 and a generator housing 722 coupled to the patch 721. The stimulation energy generator 720 further includes: a generator circuit 724; and a proximal generator antenna 723 and a distal generator antenna 726 that are coupled to the generator circuit 724. The two generator antennas 723 and 726 are located at two sides of the generator circuit 724 and are coupled to the generator circuit 724 via respective conductive wires 725. The generator antennas 723 and 726 and the generator circuit 724 are all disposed within the generator housing 722. The use of two antennas can improve the electromagnetic coupling between the stimulation energy generator 720 and the implantable electrode assembly in the body, thereby saving power consumption of the stimulation energy generator 720 or increasing the amplitude of the stimulation signal. In some embodiments, at least one of the generator antennas 723 and 726 can be configured as a three-dimensional antenna coil, which helps adjust the strength and uniformity of the electromagnetic field to achieve maximum magnetic field strength and uniform magnetic field distribution. In this way, even if a certain longitudinal offset occurs between the stimulation energy generator 720 and the implantable electrode assembly, this can be compensated through electromagnetic field adjustment, thereby providing excellent tolerance to offset.

As shown in FIG. 7c, a stimulation energy generator 730 adopts a split configuration, and includes a patch 731 and a generator housing 732 coupled to the patch 731. A generator antenna 733 and a generator circuit 734 are disposed within the patch 731 and the generator housing 732, respectively, and are coupled to each other via a conductive wire 735. In some embodiments, the generator antenna 733 may be constructed as a three-dimensional antenna coil.

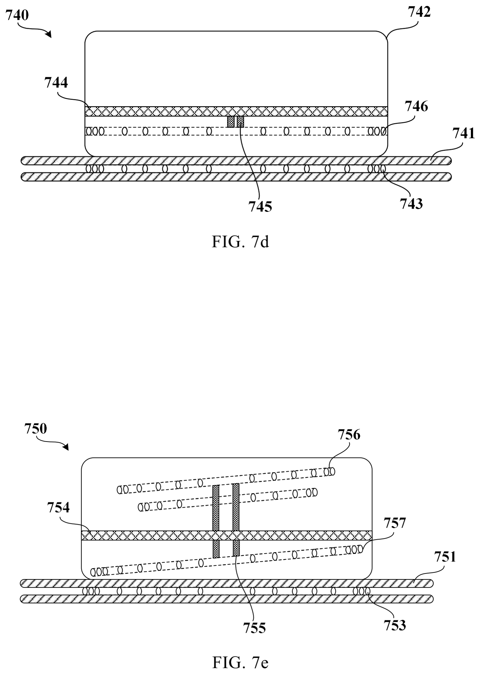

As shown in FIG. 7d, the stimulation energy generator 740 adopts a split configuration, and includes a patch 741 and a generator housing 742 coupled to the patch 741. A proximal generator antenna 743 is disposed within the patch 741 and optionally adopts a planar antenna coil configuration. Furthermore, the stimulation energy generator 740 further includes a distal generator antenna 746. The distal generator antenna 746 is disposed within the generator housing 742 along with a generator circuit 744 and is coupled to the generator circuit 744 via a conductive wire 745. In some embodiments, both the proximal generator antenna 743 and the distal generator antenna 746 can adopt a three-dimensional antenna coil configuration. In some optional embodiments, the proximal generator antenna 743, located closer to the patient's body, can serve as a relay antenna, which can receive stimulation energy in the form of an electromagnetic field emitted by the distal generator antenna 746 and transmit it to the implantable electrode assembly within the patient's body. This antenna relay configuration further enhances the flexibility of the internal structure and circuit design of the stimulation energy generator 740. In addition, when the proximal generator antenna 743 is used as a relay antenna, the patch 741 may not be electrically connected to the generator circuit 744, so that electrical losses in the interface is avoided, thereby achieving low-cost manufacturing, and the lateral offset between the distal generator antenna 746 and the electrode antenna of the implantable electrode assembly can be compensated for.

As shown in FIG. 7e, a stimulation energy generator 750 adopts a split configuration, and includes a patch 751 and a generator housing 752 coupled to the patch 751. A proximal generator antenna 753 is disposed within the patch 751 and may optionally adopt a planar antenna coil configuration. In addition, the stimulation energy generator 750 further includes two distal generator antennas 756 and a generator antenna 757. The two distal generator antennas 756 and the generator antenna 757 are disposed within the generator housing 752 along with a generator circuit 754 and are respectively positioned at two sides of the generator circuit 754. The generator antennas 756 and the generator antenna 757 are coupled to the generator circuit 754 via respective conductive wires 755, while the generator antenna 753 may or may not be coupled to the generator circuit 754. In some embodiments, the two distal generator antennas 756 and the generator antenna 757 that are located in the generator housing 752 may each adopt a three-dimensional antenna coil configuration, while the proximal generator antenna 753, located in the patch 751, may adopt a planar antenna coil configuration. In some optional embodiments, the proximal generator antenna 753, which is closer to the patient's body, can serve as a relay antenna, in which case it may not be coupled to the generator circuit 754 via a conductive wire.

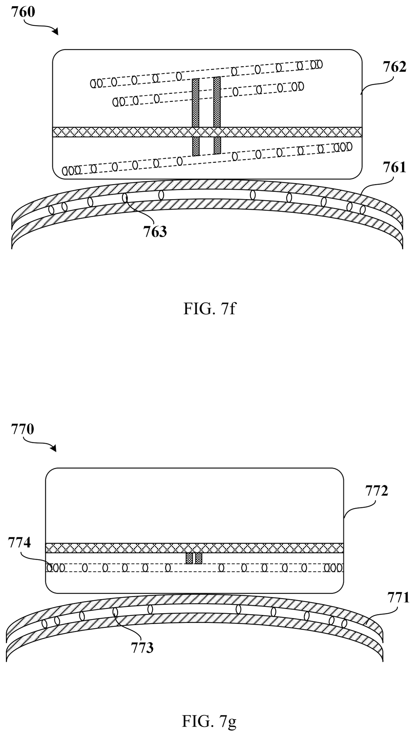

As shown in FIG. 7f, a stimulation energy generator 760 adopts a split configuration, and includes a patch 761 and a generator housing 762 coupled to the patch 761. Similar to the stimulation energy generator 750 shown in FIG. 7e, two generator antennas are disposed at two sides of the generator circuit within the generator housing 762, and can adopt a three-dimensional antenna coil configuration. However, unlike the stimulation energy generator 750 shown in FIG. 7e, the patch 761 of the stimulation energy generator 760 can be bent to a certain degree, for example, to conform to certain parts of a patient's body (e.g., the neck, legs, or other similarly curved body parts), and a generator antenna 763 located in the patch 761 can also remain in a bent state. In this way, when the stimulation energy generator 760 is worn on the patient's body, the generator antenna 763 can assume a three-dimensional antenna coil configuration, thereby enabling better alignment with the implantable electrode assembly within the patient's body.

As shown in FIG. 7g, a stimulation energy generator 770 adopts a split configuration, and includes a patch 771 and a generator housing 772 coupled to the patch 771. Similar to the stimulation energy generator 760 shown in FIG. 7f, the patch 771 of the stimulation energy generator 770 can be bent to a certain degree, for example, to conform to certain parts of a patient's body, and a generator antenna 773 located in the patch 771 can also remain in a bent state. Within the generator housing 772, a generator antenna 774 employing a planar antenna coil is disposed at a side of a generator circuit and coupled to the generator circuit.

It can be appreciated by those skilled in the art that for the generator antennas used in the stimulation energy generators 710 to 770 shown in FIGS. 7a to 7g, whether they are planar antenna coils or three-dimensional antenna coils, a uniform distribution of the electromagnetic field formed by the antennas can be enabled by designing and controlling the spacing between the turns of each antenna coil in its respective axial direction.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments only illustrate several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be subjected to the appended claims.

## Claims

1. A device for stimulating a peripheral nerve, comprising:
an implantable electrode assembly capable of being implanted in a patient's body and configured to apply a stimulation signal to a nerve; wherein the implantable electrode assembly comprises:
a stimulation electrode secured to a predetermined location of the patient's body and adjacent to the nerve in the patient's body, the stimulation electrode being configured to operably apply the stimulation signal to the nerve; and
an electrode circuit coupled to the stimulation electrode and configured to receive stimulation energy in a form of an electromagnetic field, convert the stimulation energy into the stimulation signal, and provide the stimulation signal to the stimulation electrode, wherein the stimulation energy comes from outside the implantable electrode assembly; and
a stimulation energy generator capable of being worn externally on the patient's body to provide the stimulation energy to the implantable electrode assembly via electromagnetic field coupling; wherein the stimulation energy generator comprises:
a generator circuit configured for generating the stimulation energy in the form of the electromagnetic field; and
a power supply coupled to the generator circuit and configured to supply power to the generator circuit.

2. The device according to claim 1, wherein the stimulation electrode comprises:
an electrode sleeve configured as a sheath structure having a hollow channel, wherein the electrode sleeve is provided with an opening along a circumferential direction thereof to allow the nerve to enter the electrode sleeve via the opening and be accommodated in the hollow channel of the electrode sleeve; and
an electrode contact protruded from an inner surface of the electrode sleeve to make contact with the nerve.

3. The device according to claim 2, wherein the electrode sleeve has overlapping portions in the circumferential direction thereof to allow for variations in a sectional diameter of the electrode sleeve.

4. The device according to claim 2, wherein the stimulation electrode comprises a plurality of electrode contacts, and the plurality of electrode contacts are arranged along a length direction of the electrode sleeve.

5. The device according to claim 4, wherein each of the electrode contacts in the stimulation electrode extends along the circumferential direction of the electrode sleeve.

6. The device according to claim 2, wherein the implantable electrode assembly further comprises:
a lead configured to connect the electrode sleeve and the electrode circuit to achieve coupling between the stimulation electrode and the electrode circuit.

7. The device according to claim 1, wherein the implantable electrode assembly further comprises:
an electrode circuit housing defining a cavity configured for accommodating the electrode circuit.

8. The device according to claim 7, wherein the electrode circuit housing further comprises:
a housing body having an opening; and
a feedthrough cover connected to the housing body at the opening, wherein the feedthrough cover is provided with a through hole to allow the electrode circuit to be led out from the electrode circuit housing.

9. The device according to claim 8, wherein the housing body has an elongated shape, wherein the housing body has a metal ring on a side where the opening is located.

10. The device according to claim 7, wherein the implantable electrode assembly further comprises:
an anchoring member configured to secure the electrode circuit housing inside the patient's body, wherein the anchoring member comprises a butterfly clasp, which is configured to secure the electrode circuit housing to bones or muscles around a predetermined location of the patient's body.

11. The device according to claim 7, wherein the implantable electrode assembly further comprises:
an anchoring member configured to secure the electrode circuit housing inside the patient's body, wherein the anchoring member comprises a binding wire configured to secure the electrode circuit housing to muscles around a predetermined location of the patient's body.

12. The device according to claim 1, wherein the stimulation electrode comprises a plurality of electrode contacts, and the stimulation energy generator is further configured to provide the stimulation signal in a form of pulse to the plurality of electrode contacts sequentially or simultaneously via the electrode circuit.

13. The device according to claim 12, wherein a maximum voltage amplitude of the stimulation signal in the form of pulse is in a range of 3 V to 12 V, a pulse width is in a range of 30 us to 240 us, and a pulse frequency is in a range of 0.1 Hz to 50 Hz.

14. The device according to claim 1, wherein the stimulation energy generator further comprises:
an indicator member, the stimulation energy generator being further configured to indicate alignment between the stimulation energy generator and the implantable electrode assembly when the stimulation energy generator is aligned with the implantable electrode assembly.

15. The device according to claim 1, further comprising:
a sensing element configured to sense an abnormal state of the patient's body and generate a corresponding indication signal.

16. The device according to claim 1, wherein the electrode circuit comprises an electrode antenna.

17. The device according to claim 1, further comprising:
an extracorporal controller configured to adjust the stimulation energy and provide the adjusted stimulation energy to the implantable electrode assembly via the electromagnetic field coupling.

18. The device according to claim 1, wherein the stimulation energy generator further comprises:
a generator housing configured to accommodate at least a portion of the generator circuit and the power supply; and
a patch removably coupled to the generator housing.

19. The device according to claim 18, wherein the generator circuit comprises a generator antenna disposed in the patch.

20. The device according to claim 18, wherein the patch comprises:
a connecting member configured to removably couple to the generator housing.

21. The device according to claim 20, wherein the generator housing comprises:
a generator housing upper portion and a generator housing lower portion that collectively define a cavity configured for accommodating at least a portion of the generator circuit and the power supply.

22. The device according to claim 18, wherein the generator circuit comprises:
a generator antenna disposed in the generator housing.

23. The device according to claim 18, wherein the generator circuit comprises:
a plurality of generator antennas, wherein at least one of the plurality of generator antennas is disposed in the generator housing, and another of the plurality of generator antennas is disposed in the patch; and the generator antenna in the patch is configured to relay an electromagnetic field coupling between the at least one of the generator antennas in the generator housing and an electrode antenna in the implantable electrode assembly.

24. The device according to claim 1, wherein the implantable electrode assembly further comprises:
a housing body defining a cavity configured for accommodating the electrode circuit; and
wherein the stimulation electrode comprises:
an electrode sleeve attached to the housing body and configured as a sheath structure having a hollow channel, wherein the electrode sleeve has an opening along a circumferential direction thereof to allow the nerve to enter the electrode sleeve via the opening and be accommodated in the hollow channel of the electrode sleeve, and
an electrode contact protruded from an inner surface of the electrode sleeve to make contact with the nerve.
